# EUROPEAN PATENT SPECIFICATION

(11) **EP 3 004 846 B1**
(45) Date of publication and mention of the grant of the patent: **02.08.2023**
(21) Application number: 14723887.7
(22) Date of filing: 17.04.2014
(51) Int. Cl.: G01N 21/64

(54) **A KIT FOR DETECTING MICRO-RNA EXTRACTED FROM A SAMLPLE OF BODY FLUID AS WELL AND A METHOD FOR THE DETECTION THEREOF**
KIT FÜR DEN NACHWEIS VON AUS EINER KÖRPERFLÜSSIGKEITSPROBE EXTRAHIERTER MIKRO-RNA UND NACHWEISVERFAHREN DAFÜR
TROUSSE PERMETTANT DE DÉTECTER UN MICRO-ARN EXTRAIT D'UN ÉCHANTILLON DE FLUIDE CORPOREL ET PROCÉDÉ POUR SA DÉTECTION

(30) Priority: 29.05.2013 IT VR20130132
(43) Date of publication of application: 13.04.2016
(73) Proprietor: Optoelettronica Italia S.R.L., 38121 Trento (IT)
(72) Inventor: MAGLIONE, Alfredo, 38123 Trento (TN) (IT); RESS, Cristina, 38030 Ceola di Giovo (IT)
(74) Representative: Gallo, Luca
(86) International application number: PCT/IB2014/060793
(87) International publication number: WO 2014/191850

(56) References cited:
- SUE RUTHERFORD SIEGEL ET AL: "Circulating microRNAs involved in multiple sclerosis", MOLECULAR BIOLOGY REPORTS ; AN INTERNATIONAL JOURNAL ON MOLECULAR AND CELLULAR BIOLOGY, KLUWER ACADEMIC PUBLISHERS, DO, vol. 39, no. 5, 10 January 2012 (2012-01-10), pages 6219-6225, XP035033762, ISSN: 1573-4978, DOI: 10.1007/S11033-011-1441-7
- "ScanArray Express Microarray Analysis System User Manual", , 1 January 2002 (2002-01-01), XP055099277, Retrieved from the Internet: URL:http://www.bionet.nsc.ru/gep/materials /ScanArray_Express_User_Manual.pdf [retrieved on 2014-01-29]
- F. LIN ET AL: "Silicon photomultipliers for improved biomolecule detection", PROCEEDINGS OF SPIE, vol. 6092, 9 February 2006 (2006-02-09), pages 60920L-60920L-7, XP055099086, ISSN: 0277-786X, DOI: 10.1117/12.644466
- "Yakima Silicon Photomultiplier", , 1 June 2012 (2012-06-01), XP055099095, Retrieved from the Internet: URL:http://voxtel-inc.com/files/2012/07/Ya kima_LF_Datasheet_SiPM_5_2012.pdf [retrieved on 2014-01-29]

## Description

The present invention regards a kit for the optical detection of a micro- RNA of interest in a sample extracted from a body fluid. The present invention also regards a method for detecting such micro- RNA of interest. As is known, there is increasing interest in the possibility to make an early prediction of pathologies with dire prognoses by means of examinations that are welldirected, precise, possibly little-invasive as well as inexpensive.

For such purpose, micro-RNA are considered very promising molecules in the diagnostic field, even if they are present in body fluids with low concentration. These are small, non-codifying RNA molecules (about 21-23 nucleotides), which mediate the post-transcriptional regulation of the gene expression, acting as master switches on the human genome. There have been numerous studies which attest how such molecules exert a key role on genes specifically involved in some pathologies (e.g. cancer, cardiovascular diseases, liver diseases, neurological diseases etc.), and in processes such as differentiation, programmed cellular death and tumor transformation. It is therefore clearly important to provide sensitive and reliable techniques for the detection of such molecules, extracted from a sample of a body fluid.

Most of the methods for detecting a micro-RNA of interest extracted from a sample of body fluid, e.g. blood (serum and/or plasma), urine or saliva, are based on the hybridization principle, in which each molecule of micro-RNA of interest (also called "target" in jargon), present in a sample C under examination, interacts ("hybridizes" in technical jargon) with a respective complementary oligonucleotide synthetic probe with single strand (also called "probe" in jargon). In the hybridization, the chain of nucleotide bases, which constitute an oligonucleotide probe, is linked in a specific manner to the complementary sequence of nucleotides present in a strand of target micro-RNA, thus giving rise to a molecule with double strand. The hybridization event indicates the presence of a specific micro-RNA contained in the sample C under examination.

Once such hybridization has occurred, the detection methods of the prior art provide for the actual measurement of the quantity of the target micro-RNA thus hybridized, contained in the sample C under examination, by means of the generation of a measurable signal obtained, e. g. electrochemically (by means of redox reactions) or optically due to fluorescence or bioluminescence or by means of autoradiography, generatable by a molecular marker (called "tag", "label" or "reporter" in jargon) that can be conjugated to the hybridized micro-RNA of the sample or to the oligonucleotide probe or as an intercalating agent inserted in the double strand or in any case associable with the hybridization event between the two complementary strands.

As is known, both the electrochemical approach and the optical fluorescence or bioluminescence approach can be conducted in solid state or in solution, depending on whether the oligonucleotide probe specific for the recognition of the complementary micro-RNA has been previously constrained to the surface on which the hybridization occurs (e.g. comprising microparticles or nanoparticles or a flat surface) or is found in solution (suspended in a liquid). For such purpose, various devices and methods were developed over the years for measuring micro-RNA, including for example the microarray or deep sequencing, or other biochemical methods such as Northern blotting, quantitative reverse- transcriptase-PCR (qRT-PCR) or in situ hybridization (ISH).

The devices and the methods mentioned briefly above suffer from several drawbacks. One of these is poor sensitivity. Indeed, given the low concentrations of micro-RNA in the body fluids, they are unsuitable for obtaining a reliable direct reading of the quantity of a micro-RNA of interest present in the sample under examination and therefore require a step of amplification of the micro-RNA of interest in the sample C to be analyzed by means of enzymatic reaction (PCR). Such preliminary step, in addition to being arduous and costly, lengthens the analysis times of the sample itself. The average times of amplification by means of PCR of the micro-RNA of interest in the sample C to be analyzed can even reach 2 hours. Overall, therefore, the execution of such methods typically requires a time greater than 2 hours.

Document Sue Rutherford Siegel et al. "Circulation microRNAs involved in multiple sclerosis" Molecular Biology Reports, Vol. 39, No. 5, 10 January 2012, pages 6219-6225 discloses a circulating miRNA signature involved in MS (Multiple Sclerosis) that could serve as a potential prognostic and diagnostic biomarker for MS.

Document "ScanArray Express Microarray Analysis System User Manual", 1 January 2002 (2002-01-01-), describes a sensitive microarray laser scanner with software for microarray scanning and quantitation.

Document F. Lin et al. "Silicon photomultiplier for improved biomolecule detection", Proceeding Of Spie, vol. 6092, 9 February 2006, describes the use of SiPM technology for the measurement of fluorescent biomolecules.

Document "Yakima Silicon Photomultiplier", 1 June 2012, describes the Silicon Photomultiplier Series SQBF.

Therefore, the main object of the present invention is to provide a kit for the optical detection of a micro-RNA of interest in a sample extracted from a body fluid which is very sensitive, in the sense that it allows detecting very low concentrations of micro-RNA and that it requires a minimum quantity of sample to be analyzed.

Still another object of the present invention is to provide a method for the optical detection of a micro-RNA of interest extracted from a body fluid which is quicker than the conventional methods.

A further object of the present invention is to provide a method for the optical detection of a micro-RNA of interest extracted from a body fluid which is precise and reliable.

Not least object of the present invention is to provide a method for the optical detection of a micro-RNA of interest extracted from a body fluid which is practical to implement.

According to a first aspect of the present invention, a kit is provided as defined by claim 1, with particular embodiments defined by claims 2-9.

According to a further aspect of the present invention, a method is provided as defined by claim 10, with particular embodiments described by claims 11-13.

Further aspects and advantages of the present invention will be clearer from the following detailed description of a currently preferred embodiment thereof, illustrated as a merely non-limiting example in the accompanying drawings, in which:
Figure 1 illustrates a slightly from above perspective view of a kit for the optical detection of a micro-RNA of interest extracted from a body fluid;
Figure 2 shows a block diagram representing the main components of the kit of Fig.1, according to a first embodiment of the present invention;
Figure 3 is a block diagram representing the main components of the kit of Fig.1, according to an example; and
Figure 4 illustrates a variant of the example illustrated in Fig. 3.

In the accompanying drawings, equivalent or similar parts or components have been marked with the same reference numerals.

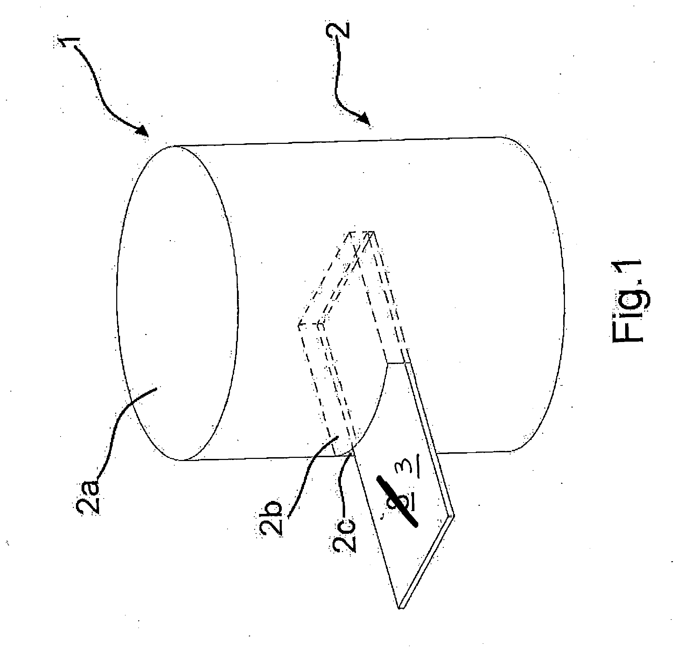

With reference now to Figures 1 to 4, it will be observed that a kit for the optical detection of a micro-RNA of interest in a sample C extracted from a body fluid, according to a first embodiment of the present invention, is generically indicated with the reference number 1 and comprises a device 2 formed by a housing casing 2a, and delimiting a housing seat 2b, accessible from the outside by means of an opening 2c obtained in the casing itself. The kit according to the present invention also comprises a container means 3 provided, in use, for containing a sample C to be analyzed, extracted from the body fluid and containing a micro-RNA of interest, of which it is desired to detect the presence in the sample. The container 3, made of a material that is permeable to light radiations, as will be better explained hereinbelow, emitted by a respective light source and by a molecular marker is for example made of transparent material and is provided insertable/disconnectable in/from the housing seat 2b.

In the housing casing 2a of the device 2 of the kit according to the present invention, an optical excitation group 5 is mounted, designed to deliver an excitation light radiation λ towards the housing seat 2b and hence towards the container 3 that, in use, is insertable in such seat.

More particularly, the optical excitation group 5 comprises a light source 5a, e.g. of LED type, suitable for delivering an excitation light radiation λ. Such light radiation λ has a wavelength in the excitation range of a molecular marker that is optically excitable and associable in a known manner with the molecules of micro-RNA of interest in the sample C under examination. The LED light source will for example be selected with emission peak around 470 nm

As will be noted, one such type of light source 5a, being fairly inexpensive, allows maintaining limited production costs as well as limited power consumption of the kit according to the present invention. In any case, other types of light sources 5a can be used, such as light sources of laser type or one or more white light sources, e.g. a halogen lamp.

The optical excitation group 5 of the kit according to the present invention also comprises collimator means 5b of such excitation light radiation λ, for example comprising a converging optical lens set to collimate the light radiation λ towards the housing seat 2b of the casing 2a of the device 2. Such collimator means 5b for example comprise an optical lens with focal length equal to about 10 mm placed at a distance equal to the focal length itself from the LED light source 5a.

Advantageously, the optical excitation group 5 according to the present invention also comprises an optical filtering means 5c between the collimator means 5b and the housing seat 2b. The optical filtering means 5c are set to limit the spectrum of the light radiation component λ, emitted by the source 5a, which reaches the housing seat 2b. The light radiation λ1 which crosses through the optical filtering means 5c has a spectrum such to obtain, in use, the optical excitation of the marker associable with the sample C under examination. The optical filtering means 5c also performs the function of reducing to a minimum the light radiation λ that is not useful for the optical excitation of the molecular marker and which is also inevitably propagated to the other components of the device, as will be better stated below.

Optionally, the optical excitation group according to the present invention comprises one or more means 5d for screening the light radiation λ, λ1 emitted by the light source 5a. The screening means 5d, also called "optical slits" in technical jargon, are provided arranged between the optical filtering means 5c and the housing seat 2b of the device 2. They make a physical barrier for a part of the light radiation λ and/or λ1 laterally delimiting an emission zone within which such radiation λ, λ1 is free to be diffused towards the housing seat 2b. The light radiation λ, λ1 thus incident at the housing seat 2b and, in use, on the container 3, has parallel beams with greater or lesser amplitude depending on the width of the emission zone delimited by the screening means 5d.

The kit for the optical detection of a micro-RNA of interest in a sample C according to the present invention also comprises a group 6 for detecting an emission light radiation A2 that is generated, in use, by the molecular marker associable with the optically excitable micro-RNA molecules of interest. Such detection group 6 is provided housable in the device 2 at the housing seat 2b. The emission light radiation A2 generated by the molecular marker is emitted by the sample C under examination in response to the emission, by the light source 5a, of the excitation light radiation A1. The emission light radiation A2 signals that the hybridization of the micro-RNA of interest with a respective oligonucleotide probe has occurred. The detection group 6 is designed to detect the light radiation A2 and to supply one or more electric output signals SO (signal output) correlated with the quantity, in the sample C, of the micro-RNA of interest.

The kit according to the present invention furthermore comprises a processing unit 7, preferably mounted in the device 2, designed to process in a known manner the electric signal or signals SO supplied by the detection group 6 and, to in turn, to output an index or measurement of such quantity of micro-RNA of interest in the examined sample C.

The detection group 6 comprises at least one sensor means of silicon photomultiplier type 6a (or SiPM), which, as is known, is extremely sensitive to low-intensity light signals. The sensor means SiPM 6a is suitable for detecting the photons emitted by the molecular marker associable with the micro-RNA of the sample C, once it has been optically excited. In output, the SiPM 6a supplies an electric signal SO. A typical SiPM sensor usable in the kit according to the present invention has a peak of sensitivity comprised between 380-480 nm.

Optionally, between the container 3 and the sensor means 6a, the detection group 6 of the kit according to the present invention provides for an optical filtering means 6b, designed to filter the light radiation A1 which inevitably reaches the sensors means 6a, even if in limited quantity.

The optical filtering means 6b is for example mounted or deposited on the surface itself of the sensor means 6a. With one such configuration, the optical path followed by the emission light radiation K2 is quite limited, with consequent increase of the sensitivity of the kit with respect to the sensitivity of conventional techniques.

In addition, it is quite clear that one such configuration with limited optical path for the radiations λ2 allows reducing the background noise coming from the surrounding environment and hence detecting the emission light radiation K2 in a more efficient manner. As will be observed, with one such configuration the size of the device 2 of the kit according to the present invention can also be quite limited, which renders the device easy to transport by an operator.

The kit according to the present invention also comprises administration means (not illustrated in the drawings) of any suitable type suitable for administrating, in the container means 3 of the sample C to be analyzed, the molecular marker and, if the hybridization is provided in liquid phase, oligonucleotide probes 4 complementary to the micro-RNA to be detected.

In the particular case in which the hybridization, between the micro-RNA of interest and the oligonucleotide probe(s) 4, is provided to occur in solid phase, the probes 4 will already be provided in the container means 3, e.g. bonded or constrained in a known manner to the internal surface of the container 3 or to the surface of micro- or nanoparticles present in the container 3 and not illustrated in the figure.

In this case, the kit according to the present invention comprises, in addition to the administration means of the sample C, administration means (also not illustrated in the drawings) of any suitable type that are designed to insert, in the container 3, at least one molecular marker associable with the plurality of oligonucleotide probes 4, complementary to the molecules of the micro-RNA of interest, and optically excitable.

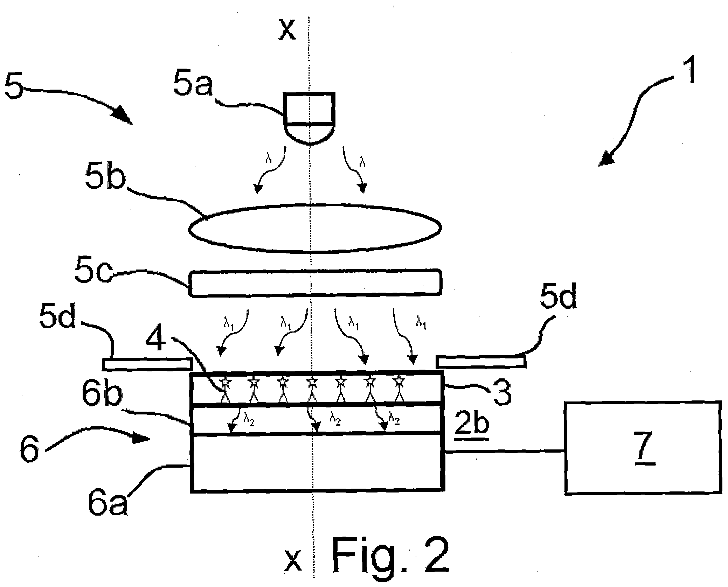

According to the first embodiment of the present invention, illustrated in Fig. 2, the kit for the optical detection of a micro-RNA of interest in a sample C extracted from a body fluid is extended along an axis x-x, passing through the housing seat 2b of the housing casing 2a of the device 2 and through the optical excitation group 5 mounted therein. More particularly, the container means 3 is advantageously supported by the detection group 6 and is integrated therewith in a disposable cartridge 8 (Fig. 1) insertable/disconnectable in/from the housing seat 2b of the housing casing 2a.

In this first embodiment, the container means 3, permeable to the light radiations λ, λ1, λ2, is directly supported by the optical filtering means 6b. The container 3 is for example made of quartz, COC, PC, PET; in use, the sample C to be analyzed extracted from the body fluid is inserted inside the container, where both the hybridization with the plurality of oligonucleotide probes 4 and the association with the molecular marker take place. In the case of hybridization in solid phase, the probe(s) 4 will already be present in the container 3. In the case of hybridization in solution, the probes can be added in a known manner together with the sample C and with the molecular marker by means of suitable administration means

With this configuration, the optical path of the emission light radiation K2 is reduced to the minimum and therefore the sensitivity of the kit is increased. The sensor means SiPM 6a can have reduced size in this case, e.g. on the order of 1x1 mm or 2x2 mm.

As an alternative, the detection group 6 can be provided anchored in the device 2, directed towards the housing seat, and the container means 3 can be provided integrated in a disposable cartridge 8 insertable/disconnectable in/from the housing seat 2b.

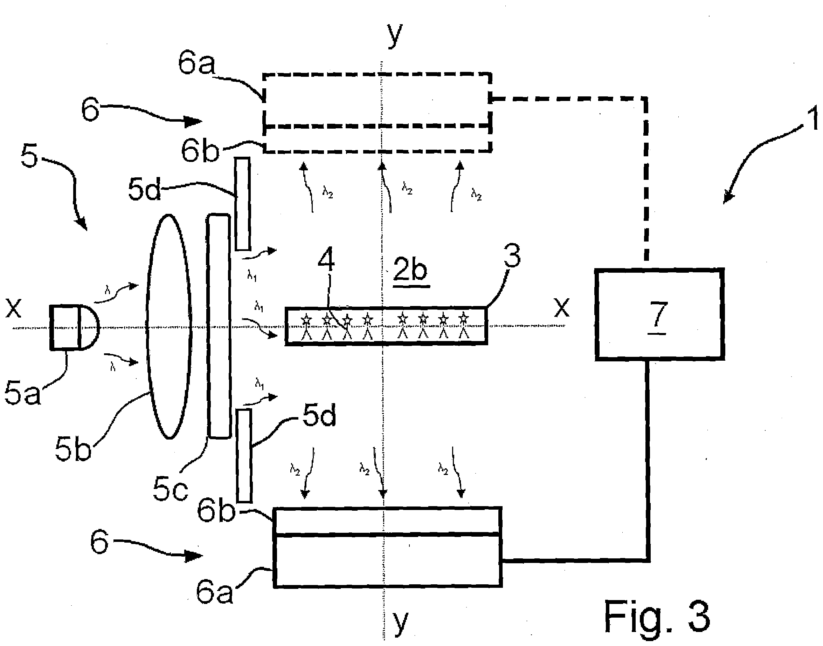

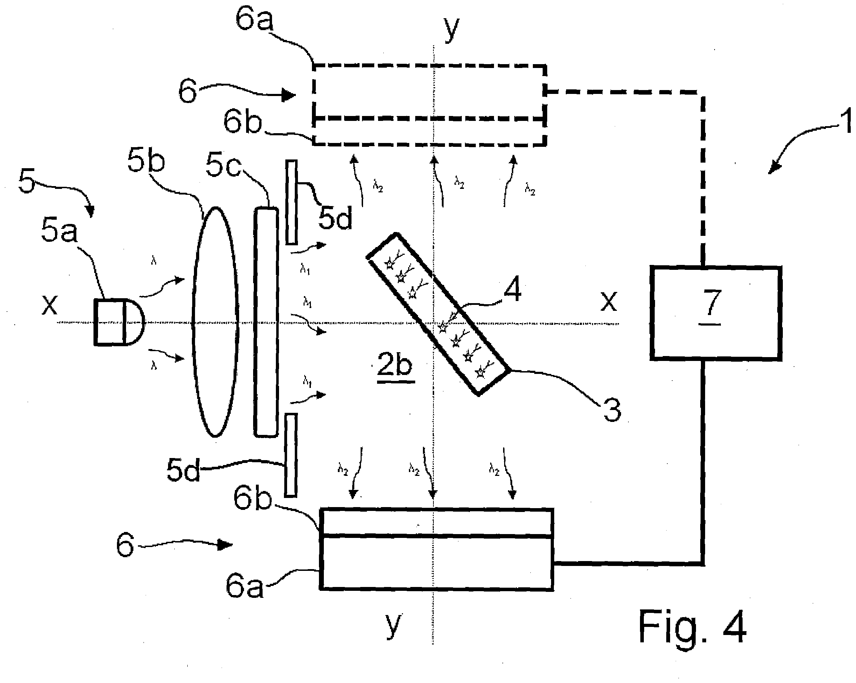

According to an example of the kit 1 (see Figures 3 and 4), the detection group 6 is housable in the device 2, shifted with respect to the axis x-x passing between the housing seat 2b and the optical excitation group 5. The detection group 6 is for example provided anchored to the housing casing 2a angularly shifted by about 90 degrees with respect to the axis x-x.

This configuration is advantageous since it allows drastically reducing the background noise caused by the excitation light radiation λ emitted by the light source 5a. Such light radiation, as stated above, is never fully eliminated even if filtered by the optical filtering means 6b of the detection group 6, and given that it is detected (even minimally) by the sensor means SiPM 6a, it actually represents the lower limit of detection of the light radiation K2 emitted by the sample C under examination. According to a first variant of the example of Fig.3, the container means 3 is housable in the housing seat 2b along the axis x-x and in such seat it laterally receives the excitation light radiation λ1.

In this case (Fig. 3), the single container means 3 is provided supportable by a suitable support to form a disposable cartridge 8 insertable/disconnectable in/from the housing seat 2b of the casing 2a, whereas the detection group 6 is provided housed in the casing itself, along a respective housing axis y-y angularly shifted about 90 degrees with respect to the axis x-x.

The sensor means SiPM 6a can have limited size, e.g. on the order of 1x1 mm or 2x2 mm.

According to a variant of such example, see Figure 4, the container 3 is provided housable in the housing seat of the casing 2a angularly shifted with respect to the axis x-x in a manner so as to be directed towards the light source 5a of the optical excitation group 5 as well as towards the sensor means 6a of the detection group 6. The container 3 is provided, also according to this variant, integratable in a disposable cartridge insertable/disconnectable in/from the housing seat 2b.

This example of Figure 4 allows an improved lighting of the sample C by the light source 5a, at the same time ensuring a reduction of the background noise caused by the same. The detection group 6 of the kit according to the present invention comprises, in this second embodiment, an optical sensor means SiPM 6a with large size, e.g. 4x4 mm.

According to a further variant of the example of Fig. 3, the detection group 6 comprises a plurality of sensor means SiPM 6a, illustrated with a dashed line in Figures 3 and 4, designed to detect the emission light radiation K2 emitted by the molecular marker associated with the micro-RNA of interest, when optically excited. Such excitation light radiation λ2, as is known, is spatially emitted in all directions and is therefore detectable by multiple sensor means 6a, provided mounted at the housing seat 2b and directed towards such seat.

Advantageously, the kit according to examples of Figures 3 and 4 provides for two sensor means 6a arranged on opposite sides with respect to the housing seat of the device 2b. With one such configuration, in addition to obtaining a double signal SO emitted by the detection group 6 (one emitted by each sensor means), the disturbance caused by the excitation light radiation λ1 is limited.

The man skilled in the art can easily understand that other configurations of the sensor means are possible. Thus, for example, the detection group 6 can also comprise a plurality of sensor means SiPM 6a arranged, in a plan orthogonal to the axis x-x, angularly offset from each other around the housing seat 2b to form a ring of sensors. In this case, the size of the sensor means SiPM can have various dimensions, e.g. ranging from 1x1 mm to 4x4 mm, depending on the number of sensors mounted in the device.

The processing group 7 of the kit according to the present invention comprises known components suitable for receiving and processing the signal(s) SO emitted by the sensor means 6a of the detection group 6. For example, it comprises signal amplifier means, set for amplifying the signal(s) SO emitted by the SiPM 6a. Such signal amplifier means comprise, for example, low-noise operational amplifiers. The processing group 7 then comprises, for example, integrator means and analog-digital converters, designed to convert the analog current signal SO into a corresponding electric voltage level which is actually an index corresponding to the quantity of micro-RNA of interest in the analyzed sample. Optionally, the index to output from the processing unit 7 can be correlated with the concentration of the micro-RNA of interest in the examined sample C.

The method for detecting a micro-RNA of interest in a sample C extracted from a body fluid is very practical in execution and reliable.

Initially, such method provides for arranging a kit as described above and arranging a sample C to be examined in the container 3 of the kit.

If the hybridization of the micro-RNA of interest in the sample C under examination occurs in solution, the method provides for administering, together with the sample C, one or more oligonucleotide probes that are complementary to the micro-RNA of interest in order to allow the formation of double-strand hybridized molecules, as well as molecule markers associable with the hybridization event.

If instead the hybridization occurs in solid phase, the probe(s) 4 will already be arranged in the container 3 and it will suffice to wait a certain time period (a few minutes) in order to allow the hybridization to take place; in a subsequent step, an optically excitable molecular marker will be added to the sample C thus hybridized, by means of the above-described administration means, and such marker will be bonded to the hybridized micro-RNA molecules of interest.

The detection method according to the present invention then provides for a step for activating the optical excitation group 5, during which the sample C is optically excited, and then a step for detecting the emission light radiation K2 emitted from the molecular marker bonded to the hybridized micro-RNA molecules of the sample C and correlated with the quantity of such micro-RNA of interest in the sample itself.

In this detection step, the sensor means SiPM 6a of the detection group 6 detect the emission light radiation K2 and each means emit, in response to such radiation, one or more electric signals SO.

The above-described method also comprises, between the step of administering the molecular marker and the step of activating the optical excitation group 5, a step for the manual insertion of the container 3 in the housing seat 2b of the device 2, integrated or not integrated in the disposable cartridge 8 depending on the case.

According to a variant of the above-described method, the step for administering the sample C can occur in an automatic manner.

Preliminary tests have demonstrated that the use of the hybridization method (which uses probes constituted by PNA oligomers and nucleobases joined with fluorophore) sold by DestiNA Genomics and the subject matter of the international application WO 2009/037473 allows increasing the selectivity of the kit according to the present invention, hence making it even more precise and reliable in the executing of the invention.

The limited times for executing the method (several minutes for the hybridization and several seconds for the optical excitation of the sample and the analysis of the corresponding signal(s) SO), given that no preliminary amplification step is necessary for the sample C to be analyzed, as well as the limited size of all the components of the kit, make it practical in use and reliable. The above-described kit and method are susceptible to numerous modifications and variants within the protection scope defined by the following claims.

The kit according to Fig. 2 comprises the container 3, at least one sensor means SiPM 6a and, if desired, an optical filtering means 6b, integrated in a disposable cartridge 8 insertable/disconnectable in/from the housing seat 2b of the device 2. Moreover, since the container 3 of the kit according to the present invention is integrated with a respective detection group 6, it can be provided lacking the wall thereof in contact with such detection group 6, such that the sample C of body fluid to be analyzed together with the oligonucleotide probes 4 and with the molecular marker will be directly in contact with the sensor means SiPM 6a or, if provided, with the optical filtering means 6b.

Furthermore, the method for detecting a micro-RNA of interest described above can be made even more precise and reliable by means of a sequential control of the turning on/turning off or activation-deactivation of the various components of the kit.

For example, it can thus be provided to activate the optical excitation group 5, so as to induce the emission by the sample C under examination of the respective emission light radiation λ2, and to deactivate such optical excitation group 5 before activating the detector group(s) 6 of the device 2.

In this manner, the light radiation λ, λ1 emitted by the optical excitation group 5 towards the container 3 will be nearly fully eliminated, and therefore the light radiation emitted by the group 5 and effectively detected by the detection group(s) 6, will comprise only the emission light radiation K2 correlated with the quantity of micro-RNA of interest contained therein.

## Claims

1. A kit for detecting a micro-RNA of interest in at least one sample (C) extracted from a body fluid, comprising:
- at least one device (2) including a housing casing (2a) in which at least one housing seat (2b) is obtained for said at least one sample (C), and at least one opening (2c) through which said housing seat (2b) is accessible from the outside;
- at least one container means (3) for said at least one sample (C), said at least one container means (3) being insertable/disconnectable in/from said housing seat (2b) through said at least one opening (2c);
- means for administering at least one molecular marker in said at least one container means (3), said at least one molecular marker being associable with said micro-RNA of interest in said sample (C);
- at least one oligonucleotide probe (4), each oligonucleotide probe (4) being set to establish a specific bond with a respective complementary sequence of nucleotides present in a strand of said micro-RNA of interest, said at least one oligonucleotide probe (4) provided in said administration means or in said at least one container means (3);
- at least one optical excitation group (5), housed in said housing casing (2a), comprising at least one light source (5a) delivering a wavelength in the excitation range of said molecular marker, designed to emit at least one excitation light radiation (λ, λ1) towards the sample (C) when the sample is located in said at least one housing seat (2b);
- at least one detection group (6), designed to detect at least one emission light radiation (λ2), that can be generated, in use, from said at least one sample (C) when the sample contains said at least one molecular marker and said at least one oligonucleotide probe (4), said at least one detection group (6) being designed to supply at least one electric output signal (SOsignal output) correlated with the quantity, in said at least one sample (C), of said micro-RNA of interest;
- at least one processing unit (7) designed to receive and process said at least one electric signal (SO) and to output an index correlated with the quantity of said micro-RNA of interest in said at least one sample (C);
said at least one container means (3) being made of a material permeable to said at least one excitation light radiation (λ, λ1) and to said at least one emission light radiation (λ2) wherein said at least one group (6) for detecting said emission light radiation (λ2) comprises at least one sensor means (6a) of silicon photomultiplier type;
said at least one container means (3) and said at least one detection group (6) are integrated in a disposable cartridge (8) insertable/disconnectable in/from said housing seat (2b).

2. A kit according to claim 1, **characterized in that** said at least one light source (5a) is of a of LED type .

3. A kit according to claim 1, **characterized in that** said optical excitation group (5) comprises at least one collimator means (5b), at least one optical filtering means (5c) between said at least one collimator means (5b) and said at least one housing seat (2b), and at least one means (5d) for screening said excitation light radiation (λ, λ1), said at least one screening means being designed to laterally delimit a zone of emission in which said excitation light radiation (λ, λ1) is free to be diffused towards said at least one housing seat (2b).

4. A kit according to claim 1, **characterized in that** said at least one group (6) for detecting said at least one emission light radiation (λ2) from said at least one sample (C) comprises at least one optical filtering means (6b), between said at least one housing seat (2b) and said at least one silicon photomultiplier sensor means (6a).

5. A kit according to claim 1, **characterized in that** said at least one detection group (6) is, in use, provided housed in said device (2) along an axis (x-x) passing between said housing seat (2b) of said device (2) and said optical excitation group (5).

6. A kit according to claim 1, **characterized in that** said at least one detection group (6) is provided housed in said housing casing (2a) of said housing (2) along a housing axis (y-y) thereof, said axis (y-y) being non-aligned, with respect to axis (x-x) passing between said housing seat (2b) of said device (2) and said optical excitation group (5), by about 90 degrees with respect to said axis (x-x); said at least one container means (3) being housed in said housing seat (2b) of said casing (2a) along said housing axis (x-x), whereby such that said excitation light radiation (λ1) hitting it laterally.

7. A kit according to claim 1, **characterized in that** said index in output from said at least one processing unit (7) is correlated with the concentration, in said at least one sample (C), of said micro-RNA of interest.

8. A kit according to claim 1, **characterized in that** the means for administering comprises automatic means for administering said at least one sample (C) in said at least one container means (3).

9. A kit according to claim 4, **characterized in that** said at least one container means (3) lacks a wall thereof in contact with said at least one detection group (6) whereby said at least one sample (C) of said body fluid to be analyzed being in direct contact with said at least one sensor means (6a) or with said at least one optical filtering means (6b).

10. A method of detecting a micro-RNA of interest in a sample (C) extracted from a body fluid, comprising the following operative steps:
- providing a kit according to claim 1;
- providing said at least one sample (C) to be examined in said at least one container means (3);
- supplying, in said at least one container means (3), at least one oligonucleotide probe (4), each oligonucleotide probe (4) being set to establish a specific bond with a respective complementary sequence of nucleotides present in a strand of said micro-RNA of interest;
- supplying, in said at least one container means (3), said at least one molecular marker associable with said micro-RNA of interest in said sample (C);
- inserting said at least one container (3) in said housing seat of said device (2);
- activating said at least one optical excitation group (5);
- activating said at least one detection group (6);
- detecting, by means of said at least one detection group (6) thus activated, said emission light radiation (λ2) coming from said at least one container means (3) and correlated with the quantity of said micro-RNA of interest in said sample (C) and
- applying at least one electric output signal (SO) correlated with said quantity, in said sample (C), of said micro-RNA of interest.).

11. A method according to claim 10, **characterized in that** said step of arranging said at least one sample (C) in said at least one container means (3), said step of supplying at least one oligonucleotide probe (4) in said at least one container means (3), and said step of supplying, in said at least one container means (3), said at least one molecular marker associable with said micro-RNA of interest occur simultaneously.

12. A method according to claim 10, **characterized in that** said step of supplying at least one oligonucleotide probe (4) in said at least one container means (3), each oligonucleotide probe (4) being set to establish a specific bond with a respective complementary sequence of nucleotides comprised in a strand of said micro-RNA of interest, occurs before said step of arranging said at least one sample (C) to be examined in said at least one container means (3).

13. A method according to claim 10, **characterized in that** between said step of activating said at least one optical excitation group (5) and said step of detecting said emission light radiation (λ2) coming from said at least one container means (3) and correlated to the quantity of said micro-RNA of interest in said sample (C), a step is provided for deactivating said at least one optical excitation group (5) before said step of activating said at least one detection group (6).

## Patentansprüche

1. Kit für den Nachweis von aus mindestens einer Körperflüssigkeitsprobe (C) extrahierter Mikro-RNA von Interesse, umfassend:
- mindestens eine Vorrichtung (2), die ein Aufnahmebehältnis (2a) enthält, in dem sich mindestens ein Aufnahmesitz (2b) für die genannte mindestens eine Probe (C) befindet, und mindestens eine Öffnung (2c), durch die der genannte Aufnahmesitz (2b) von außen zugänglich ist;
- mindestens ein Behältermittel (3) für die genannte mindestens eine Probe (C), wobei das genannte mindestens eine Behältermittel (3) in den genannten Aufnahmesitz (2b) durch die genannte mindestens eine Öffnung (2c) eingesetzt/aus diesem entnommen werden kann;
- Eingabemittel von mindestens einem molekularen Marker in das genannte mindestens eine Behältermittel (3), wobei der genannte mindestens eine molekulare Marker kann mit der genannten Mikro-RNA von Interesse in der genannten Probe (C) verbunden werden kann;
- mindestens eine Oligonukleotid-Sonde (4), wobei jede Oligonukleotid-Sonde (4) vorgesehen ist, um eine spezifische Bindung mit einer entsprechenden komplementären Sequenz von in einem Strang der genannten Mikro-RNA von Interesse vorhandenen Nukleotiden zu bilden, wobei die genannte mindestens eine Oligonukleotid-Sonde (4) in den genannten Eingabemitteln oder im genannten mindestens einen Behältermittel (3) bereitgestellt ist;
- mindestens eine optische Anregungsgruppe (5), die im genannten Aufnahmebehältnis (2a) untergebracht ist, die mindestens eine Lichtquelle (5a) umfasst, die eine Wellenlänge im Anregungsintervall des genannten molekularen Markers erzeugt, die vorgesehen ist, um mindestens eine Anregungs-Lichtstrahlung (λ, λ1) zur Probe (C) zu erzeugen, wenn die Probe im genannten mindestens einen Aufnahmesitz (2b) positioniert ist;
- mindestens eine Erfassungsgruppe (6), die vorgesehen ist, um mindestens eine Emissions-Lichtstrahlung (λ2) zu erfassen, die beim Betrieb aus der genannten mindestens einen Probe (C) erzeugbar ist, wenn die Probe den genannten mindestens einen molekularen Marker und die genannte mindestens eine Oligonukleotid-Sonde (4) enthält, wobei die genannte mindestens eine Erfassungsgruppe (6) vorgesehen ist, um mindestens ein elektrisches Ausgangssignal (SO- signal output) abzugeben, das mit der Menge in der genannten mindestens einen Probe (C) der genannten Mikro-RNA von Interesse verbunden ist;
- mindestens eine Verarbeitungseinheit (7), die vorgesehen ist, um mindestens ein elektrisches Signal (SO) zu verarbeiten und am Ausgang einen Index zu erzeugen, der mit der Menge der genannten Mikro-RNA von Interesse in der genannten mindestens einen Probe (C) verbunden ist;
wobei das genannte mindestens eine Behältermittel (3) aus einem durchlässigen Material für die genannte mindestens eine Anregungs-Lichtstrahlung (λ, λ1) und die genannte mindestens eine Emissions-Lichtstrahlung (λ2) besteht, wobei die genannte mindestens eine Erfassungsgruppe (6) der genannten Emissions-Lichtstrahlung (λ2) mindestens ein Silizium-Photomultiplier-Sensormittel (6a) umfasst,
wobei das genannte mindestens eine Behältermittel (3) und die genannte mindestens eine Erfassungsgruppe (6) in mindestens einer Einwegpatrone (8) eingebaut sind, die in den genannten Aufnahmesitz (2b) eingesetzt/aus diesem entnommen werden kann.

2. Kit nach Anspruch 1, **dadurch gekennzeichnet, dass** die genannte mindestens eine Lichtquelle (5a) eine LED-Lichtquelle ist.

3. Kit nach Anspruch 1, **dadurch gekennzeichnet, dass** die genannte optische Anregungsgruppe (5) mindestens ein Kollimatormittel (5b), mindestens ein optisches Filtrationsmittel (5c) zwischen dem genannten mindestens einen Kollimatormittel (5b) und dem genannten mindestens einen Aufnahmesitz (2b), und mindestens ein Abschirmungsmittel (5d) der genannten Anregungs-Lichtstrahlung (λ, λ1) umfasst, wobei das genannte mindestens eine Abschirmungsmittel vorgesehen ist, um einen Emissionsbereich, in dem die genannte Anregungs-Lichtstrahlung (λ, λ1) frei ist, sich zum genannten mindestens einen Aufnahmesitz zu verbreiten (2b), seitlich zu begrenzen.

4. Kit nach Anspruch 1, **dadurch gekennzeichnet, dass** die genannte mindestens eine Erfassungsgruppe (6) der genannten mindestens einen Emissions-Lichtstrahlung (λ2) von der genannten mindestens einen Probe (C) mindestens ein optisches Filtrationsmittel (6b) zwischen dem genannten mindestens einen Aufnahmesitz (2b) und dem genannten mindestens einem Silizium-Photomultiplier-Sensormittel (6a) umfasst.

5. Kit nach Anspruch 1, **dadurch gekennzeichnet, dass** die genannte mindestens eine Erfassungsgruppe (6) beim Betrieb in der genannten Vorrichtung (2) entlang einer zwischen dem genannten Aufnahmesitz (2b) der genannten Vorrichtung (2) und der genannten optischen Anregungsgruppe (5) verlaufenden Achse (x-x) untergebracht vorgesehen ist.

6. Kit nach Anspruch 1, **dadurch gekennzeichnet, dass** die genannte mindestens eine Erfassungsgruppe (6) im genannten Aufnahmebehältnis (2a) der genannten Aufnahme (2) entlang einer eigenen Aufnahmeachse (y-y) untergebracht vorgesehen ist, wobei die genannte Achse (y-y) gegenüber einer zwischen dem genannten Aufnahmesitz (2b) der genannten Vorrichtung (2) und der genannten optischen Anregungsgruppe (5) verlaufenden Achse (x-x) um ca. 90 Grad gegenüber der genannten Achse (x-x) versetzt ist; wobei das genannte mindestens eine Behältermittel (3) im genannten Aufnahmesitz (2b) des genannten Behältnisses (2a) entlang der genannten Aufnahmeachse (x-x) untergebracht ist, sodass die genannte Anregungs-Lichtstrahlung (λ1) seitlich auf dieses trifft.

7. Kit nach Anspruch 1, **dadurch gekennzeichnet, dass** der genannte aus mindestens einer Verarbeitungseinheit (7) austretende Index mit der Konzentration, in der genannten mindestens einer Probe (C), der genannten Mikro-RNA von Interesse verbunden ist.

8. Kit nach Anspruch 1, **dadurch gekennzeichnet, dass** die Eingabemittel automatische Eingabemittel der genannten mindestens einen Probe (C) im genannten mindestens einen Behältermittel (3) umfassen.

9. Kit nach Anspruch 4, **dadurch gekennzeichnet, dass** das genannte mindestens eine Behältermittel (3) frei von einer eigenen Kontaktwand mit der genannten mindestens einen Erfassungsgruppe (6) ist, sodass die genannte mindestens eine Probe (C) der genannten zu analysierenden Körperflüssigkeit in direktem Kontakt mit dem genannten mindestens einen Sensormittel (6a) oder mit dem genannten mindestens einen optischen Filtrationsmittel (6b) steht.

10. Verfahren für den Nachweis von aus mindestens einer Körperflüssigkeitsprobe (C) extrahierter Mikro-RNA von Interesse, umfassend die folgenden Arbeitsschritte:
- Vorbereiten eines Kits nach Anspruch 1;
- Vorbereiten der genannte mindestens einer im genannten mindestens einen Behältermittel (3) zu untersuchenden Probe (C);
- Ausstatten des genannten mindestens einen Behältermittels (3) mit mindestens einer Oligonukleotid-Sonde (4), wobei jede Oligonukleotid-Sonde (4) vorgesehen ist, um eine spezifische Bindung mit einer entsprechenden komplementären Sequenz der in einem Strang der genannten Mikro-RNA von Interesse vorhandenen Nukleotiden zu bilden,
- Ausstatten des genannten mindestens einen Behältermittels (3) mit mindestens einem molekularen Marker, wobei der genannte mindestens eine molekulare Marker mit der genannten Mikro-RNA von Interesse in der genannten Probe (C) verbunden werden kann;
- Einsetzen des genannten mindestens einen Behälters (3) in den genannten Aufnahmesitz der genannten Vorrichtung (2);
- Aktivieren der genannten mindestens einen optische Anregungsgruppe (5);
- Aktivieren der genannten mindestens einen Erfassungsgruppe (6);
- Erfassen, durch die genannte mindestens eine so aktivierte Erfassungsgruppe (6), der genannten Emissions-Lichtstrahlung (λ2), die vom genannten mindestens einen Behältermittel (3) ausgeht und die mit der Menge der genannten Mikro-RNA von Interesse in der genannten Probe (C) verbunden ist, und
- Anwenden mindestens eines elektrischen Ausgangssignals (SO), das mit der genannten Menge der genannten Mikro-RNA von Interesse in der genannten Probe (C) verbunden ist.

11. Verfahren nach Anspruch 10, **dadurch gekennzeichnet, dass** der genannte Schritt, die genannte mindestens eine Probe (C) im genannten mindestens einem Behältermittel (3) unterzubringen, der genannte Schritt, das genannte mindestens eine Behältermittel (3) mit mindestens einer Oligonukleotid-Sonde (4) auszustatten, und der genannte Schritt, das genannte mindestens eine Behältermittel (3) mit mindestens einem molekularen Marker auszustatten, der mit der genannten Mikro-RNA von Interesse verbunden ist, gleichzeitig erfolgen.

12. Verfahren nach Anspruch 10, **dadurch gekennzeichnet, dass** der genannte Schritt, das genannte Behältermittel (3) mit mindestens einer Oligonukleotid-Sonde (4) auszustatten, wobei jede Oligonukleotid-Sonde (4) vorgesehen ist, um eine spezifische Bindung mit einer entsprechenden komplementären Sequenz der in einem Strang der genannten Mikro-RNA von Interesse vorhandenen Nukleotiden zu bilden, vor dem genannten Schritt, die genannte mindestens eine im genannten mindestens einen Behältermittel (3) zu untersuchende Probe (C) unterzubringen, erfolgt.

13. Verfahren nach Anspruch 10, **dadurch gekennzeichnet, dass** zwischen dem genannten Schritt der Aktivierung von der genannten mindestens einen optischen Anregungsgruppe (5) und dem genannten Schritt der Erfassung der genannten Emissions-Lichtstrahlung (λ2), die vom genannten mindestens einen Behältermittel (3) ausgeht und die mit der Menge der genannten Mikro-RNA von Interesse in der genannten Probe (C) verbunden ist, ein Schritt der Deaktivierung der genannten mindestens einen optischen Anregungsgruppe (5) vor dem genannten Schritt der Aktivierung der genannten mindestens einen Erfassungsgruppe (6) vorgesehen ist.

## Revendications

1. Trousse permettant de détecter un micro-ARN présentant un intérêt dans au moins un échantillon (C) extrait d'un fluide corporel, comprenant :
- au moins un dispositif (2) incluant un boîtier (2a) dans lequel se trouve au moins un espace (2b) pour ledit au moins un échantillon (C) et au moins une ouverture (2c) par l'intermédiaire de laquelle ledit espace (2b) est accessible de l'extérieur ;
- au moins un moyen conteneur (3) pour ledit au moins un échantillon (C), ledit au moins un moyen conteneur (3) pouvant être inséré/extrait dans le/dudit espace (2b) à travers ladite au moins une ouverture (2c) ;
- des moyens d'administration d'au moins un marqueur moléculaire dans ledit au moins un moyen conteneur (3), ledit au moins un marqueur moléculaire pouvant être associé audit micro-ARN présentant un intérêt dans ledit échantillon (C) ;
- au moins une sonde oligonucléotidique (4), chaque sonde oligonucléotidique (4) étant destinée à établir un lien spécifique avec une séquence complémentaire respective de nucléotides présents dans un brin dudit micro-ARN présentant un intérêt, ladite au moins une sonde oligonucléotidique (4) étant prévue dans lesdits moyens d'administration ou dans ledit au moins un moyen conteneur (3) ;
- au moins un groupe d'excitation optique (5), situé dans ledit boîtier (2a), comprenant au moins une source lumineuse (5a) qui fournit une longueur d'onde dans la plage d'excitation dudit marqueur moléculaire, destinée à émettre au moins un rayonnement lumineux d'excitation (λ, λ1) vers l'échantillon (C) lorsque celui-ci est placé dans ledit au moins un espace (2b) ;
- au moins un groupe de détection (6), destiné à détecter au moins un rayonnement lumineux d'émission (λ2), pouvant être généré, en cours d'utilisation, par ledit au moins un échantillon (C) lorsque celui-ci contient ledit au moins un marqueur moléculaire et ladite au moins une sonde oligonucléotidique (4), ledit au moins un groupe de détection (6) étant destiné à fournir au moins un signal électrique de sortie (SO- signal output) lié à la quantité, dans ledit au moins un échantillon (C), dudit micro-ARN présentant un intérêt ;
- au moins une unité de traitement (7) destinée à recevoir et à traiter ledit au moins un signal électrique (SO) et à fournir en sortie un indice liée à la quantité dudit micro-ARN présentant un intérêt dans ledit au moins un échantillon (C) ;
ledit au moins un moyen conteneur (3) étant constitué d'un matériau perméable audit au moins un rayonnement lumineux d'excitation (λ, λ1) et audit au moins un rayonnement lumineux d'émission (λ2), où ledit au moins un groupe de détection (6) dudit rayonnement lumineux d'émission (λ2) comprend au moins un moyen capteur (6a) de type photomultiplicateur au silicium ;
ledit au moins un moyen conteneur (3) et ledit au moins un groupe de détection (6) sont intégrés dans une cartouche jetable (8) pouvant être insérée/extraite dans le/dudit espace (2b).

2. Trousse selon la revendication 1, **caractérisée en ce que** ladite au moins une source lumineuse (5a) est de type à LED.

3. Trousse selon la revendication 1, **caractérisée en ce que** ledit groupe d'excitation optique (5) comprend au moins un moyen collimateur (5b), au moins un moyen de filtrage optique (5c) entre ledit au moins un moyen collimateur (5b) et ledit au moins un espace (2b), et au moins un moyen de blindage (5d) dudit rayonnement lumineux d'excitation (λ, λ1), ledit au moins un moyen de blindage étant destiné à délimiter latéralement une zone d'émission dans laquelle ledit rayonnement lumineux d'excitation (λ, λ1) est libre de se diffuser vers ledit au moins un espace (2b).

4. Trousse selon la revendication 1, **caractérisée en ce que** ledit au moins un groupe de détection (6) dudit au moins un rayonnement lumineux d'émission (λ2) dudit au moins un échantillon (C) comprend au moins un moyen de filtrage optique (6b), entre ledit au moins un espace (2b) et ledit au moins un moyen capteur (6a) photomultiplicateur au silicium.

5. Trousse selon la revendication 1, **caractérisée en ce que** ledit au moins un groupe de détection (6) est prévu, en cours d'utilisation, dans ledit dispositif (2) le long d'un axe (x-x) passant entre ledit espace (2b) dudit dispositif (2) et ledit groupe d'excitation optique (5).

6. Trousse selon la revendication 1, **caractérisée en ce que** ledit au moins un groupe de détection (6) est prévu dans ledit boîtier (2a) dudit espace (2) le long de son propre axe d'emplacement (y-y), ledit axe (y-y) étant décalé, par rapport à un axe (x-x) passant entre ledit espace (2b) dudit dispositif (2) et ledit groupe d'excitation optique (5), d'environ 90 degrés par rapport audit axe (x-x) ; ledit au moins un moyen conteneur (3) étant placé dans ledit espace (2b) dudit boîtier (2a) le long dudit axe d'emplacement (x-x), de manière à ce que ledit rayonnement lumineux d'excitation (λ1) le frappe latéralement.

7. Trousse selon la revendication 1, **caractérisée en ce que** ledit indice sortant de ladite au moins une unité de traitement (7) est lié à la concentration, dans ledit au moins un échantillon (C), dudit micro-ARN présentant un intérêt.

8. Trousse selon la revendication 1, **caractérisée en ce que** les moyens d'administration comprennent des moyens automatiques d'administration dudit au moins un échantillon (C) dans ledit au moins un moyen conteneur (3).

9. Trousse selon la revendication 4, **caractérisée en ce que** ledit au moins un moyen conteneur (3) est dépourvu de sa propre paroi de contact avec ledit au moins un groupe de détection (6), de manière à ce que ledit au moins un échantillon (C) dudit fluide corporel à analyser soit en contact direct avec ledit au moins un moyen capteur (6a) ou avec ledit au moins un moyen de filtrage optique (6b).

10. Méthode pour détecter un micro-ARN présentant un intérêt dans un échantillon (C) extrait d'un fluide corporel, comprenant les étapes opérationnelles suivantes :
- préparer une trousse selon la revendication 1 ;
- préparer ledit au moins un échantillon (C) à examiner dans ledit au moins un moyen conteneur (3) ;
- fournir, dans ledit au moins un moyen conteneur (3), au moins une sonde oligonucléotidique (4), chaque sonde oligonucléotidique (4) étant destinée à établir un lien spécifique avec une séquence complémentaire respective de nucléotides présents dans un brin dudit micro-ARN présentant un intérêt ;
- fournir dans ledit au moins un moyen conteneur (3), ledit au moins un marqueur moléculaire pouvant être associé audit micro-ARN présentant un intérêt dans ledit échantillon (C) ;
- insérer ledit au moins un conteneur (3) dans ledit espace dudit dispositif (2) ;
- activer ledit au moins un groupe d'excitation optique (5) ;
- activer ledit au moins un groupe de détection (6) ;
- détecter, par l'intermédiaire dudit au moins un groupe de détection (6) ainsi activé, ledit rayonnement lumineux d'émission (λ2) provenant dudit au moins un moyen conteneur (3) et lié à la quantité dudit micro-ARN présentant un intérêt dans ledit échantillon (C) et
- appliquer au moins un signal électrique de sortie (SO) lié à ladite quantité, dans ledit échantillon (C), dudit micro-ARN présentant un intérêt.

11. Méthode selon la revendication 10, **caractérisée en ce que** ladite étape de disposer ledit au moins un échantillon (C) dans ledit au moins un moyen conteneur (3), ladite étape de fournir dans ledit au moins un moyen conteneur (3) au moins une sonde oligonucléotidique (4) et ladite étape de fournir, dans ledit au moins un moyen conteneur (3), ledit au moins un marqueur moléculaire associable audit micro-ARN présentant un intérêt, ont lieu simultanément.

12. Méthode selon la revendication 10, **caractérisée en ce que** ladite étape de fournir dans ledit au moins un moyen conteneur (3) au moins une sonde oligonucléotidique (4), chaque sonde oligonucléotidique (4) étant destinée à établir un lien spécifique avec une séquence complémentaire respective de nucléotides compris dans un brin dudit micro-ARN présentant un intérêt, a lieu avant ladite étape de disposer ledit au moins un échantillon (C) à examiner dans ledit au moins un moyen conteneur (3).

13. Méthode selon la revendication 10, **caractérisée en ce qu'**entre ladite étape d'activation dudit au moins un groupe d'excitation optique (5) et ladite étape de détection dudit rayonnement lumineux d'émission (λ2) provenant dudit au moins un moyen conteneur (3) et lié à la quantité dudit micro-ARN présentant un intérêt dans ledit échantillon (C), une étape de désactivation dudit au moins un groupe d'excitation optique (5) est prévue avant ladite étape d'activation dudit au moins un groupe de détection (6).
